# EUROPÄISCHE PATENTSCHRIFT

(11) **EP 3 391 045 B1**
(45) Veröffentlichungstag und Bekanntmachung des Hinweises auf die Patenterteilung: **12.02.2020**
(21) Anmeldenummer: 16820151.5
(22) Anmeldetag: 14.12.2016
(51) Int. Cl.: G01N 33/38, G01N 19/10

(54) **INDIKATORVORRICHTUNG ZUR BAUSTOFF-FEUCHTEBESTIMMUNG**
INDICATOR APPARATUS FOR DETERMINING CONSTRUCTION MATERIAL MOISTURE
DISPOSITIF INDICATEUR POUR LA DÉTERMINATION DE L'HUMIDITÉ D'UN MATÉRIAU DE CONSTRUCTION

(30) Priorität: 14.12.2015 AT 510612015
(43) Veröffentlichungstag der Anmeldung: 24.10.2018
(73) Patentinhaber: Technische Universität Wien, 1040 Wien (AT)
(72) Erfinder: KOLBITSCH, Andreas, 1090 Wien (AT); BERGER, Leopold, 3073 Stössing (AT); EICHINGER, Karl, 3143 Pyhra (AT)
(74) Vertreter: Patentanwaltskanzlei Matschnig & Forsthuber OG
(86) Internationale Anmeldenummer: PCT/AT2016/060126
(87) Internationale Veröffentlichungsnummer: WO 2017/100813

(56) Entgegenhaltungen:
- DE-T2- 69 101 158
- FR-A1- 2 840 068
- US-A- 3 117 442
- US-A1- 2015 285 730

## Beschreibung

Die Erfindung betrifft eine Feuchteindikatorvorrichtung zur hygrometrischen Bestimmung der Restfeuchte eines Baustoffs, wobei die Feuchteindikatorvorrichtung ein Gehäuse mit zumindest einem Gehäuseinnenraum umfasst, der über zumindest eine mit einer wasserdampfdurchlässigen Membran bedeckten Diffusionsöffnung mit der Gehäuseumgebung kommuniziert, wobei das Gehäuse ein Anzeigefeld aufweist.

Ein hygrometrisches Messverfahren beruht allgemein auf der hygrometrischen Feuchtemessung nach einem Feuchteausgleich zwischen der Luft und dem zu untersuchenden Material bzw. Baustoff. Jeder Baustoff nimmt aus der Umgebung Wasser auf, welches zunächst durch Sorption an den Porenoberflächen und in den Poren angelagert wird. Die Menge des aus der Luft aufgenommenen Wassers hängt vom Material des Baustoffs und von der relativen Feuchte der Luft ab. Sinkt die relative Feuchte der Luft, gibt der Baustoff durch Desorption wieder Feuchte an die Luft ab. Diese hinlänglich bekannten Vorgänge werden durch sogenannte Sorptionsisothermen beschrieben. Sind die Sorptionsisothermen bekannt, dann lässt sich aus der Luftfeuchte in einem abgeschlossenen Volumen innerhalb des Bauteils die Materialfeuchte der Umgebung angeben. Somit ist diese Messmethode nur unterhalb der Sättigungsfeuchte eines Materials einsetzbar.

Aktuell kommt es beispielsweise beim Belegen von frisch aufgebrachten Estrichen immer wieder zu Schadensfällen aufgrund von zu frühem Aufbringen eines diffusionsdichten Bodenbelags. Ebenso treten Probleme bei Gebäudewänden oder Gebäudedecken auf, die entweder nach dem Verputzen zu früh mit diffusionsdichten Wand- oder Deckenpaneelen abgedeckt werden oder welche beispielsweise im Rahmen von Sanierungsmaßnahmen zur Bauwerksentfeuchtung ebenfalls zu früh in noch zu feuchtem Zustand bereits verkleidet werden.

Je nach Baustoff bzw. abhängig von der jeweiligen individuellen Anwendung werden üblicherweise Grenzwerte der Baustofffeuchte zwischen 65% und 85%, ausgedrückt als Prozentwerte der relativen Luftfeuchte, eingestellt. Erfahrungsgemäß beträgt die Belegereife beispielsweise für einen wasserbeständigen Estrich oder Beton, welcher mit diffusionsdichten Kunststoffbelegen oder Beschichtungen bedeckt wird, etwa 75% relative Luftfeuchte. Das heißt, dass der wasserbeständige Estrich oder Beton bereits bis zu einem Feuchtegehalt entsprechend 75% relativer Luftfeuchte abgetrocknet sein muss, um belegt werden zu können. Für einen Magnesiaestrich oder Anhydritestrich, welcher mit Beschichtungen oder Belegen bedeckt wird, deren Diffusionswiderstand für Wasserdampf größer 2 m beträgt, sollte die Belegereife bei etwa 65% relativer Luftfeuchte liegen. Für Estriche oder Betonböden, die mit einem Parkettboden belegt werden, sollte die Belegereife bei etwa 55% relativer Luftfeuchte eingestellt werden. Dieser Wert von 55% relativer Luftfeuchte entspricht einer Ausgleichsfeuchte von Holz mit ca. 10 Massenprozent.

Die derzeit allgemein gebräuchlichen Nachweisverfahren zur hygrometrischen Feuchtemessung von Baumaterialien besitzen erhebliche Mängel, da diese äußerst ungenaue Ergebnisse liefern bzw. im Falle der Feuchtemessung von Estrich üblicherweise größere Mengen an Probenentnahmen erforderlich sind, welche nachteilig Schäden im Estrich verursachen. Die aktuelle Standardmethode zur Feststellung der Belegereife auf der Baustelle ist die sogenannte Calciumcarbid-Methode, abgekürzt CM-Messung. Die dabei ablaufende Reaktion von Wasser und Calciumcarbid zu Acetylen-Gas und Calciumhydroxid ist jedoch sehr ungenau. Insbesondere beim Einsatz von üblichen Trocknungsbeschleunigern, die die Abbindezeit des Estrichs verringern, oder bei Verwendung von Schnellestrichen kann die Calciumcarbid-Methode zur Feststellung der Baustofffeuchte nicht verlässlich angewendet werden.

Dieselben Nachteile sind auch für gravimetrische Methoden wie die Darr-Wäge-Trocknung zu nennen, wonach der Wassergehalt einer Materialprobe durch Gewichtsverlust beim Trocknen bestimmt wird. Sowohl bei der Darr-Wäge-Trocknung, als auch bei der CM-Methode entstehen überdies im zu untersuchenden Baustoff Probenahmelöcher von mehreren cm Durchmesser, welche jedenfalls eine anschließende Reparatur des beprobten Estrichs, Betons oder Wand- bzw. Deckenelements erforderlich machen.

Die Fachwelt arbeitet derzeit an einem Ersatz dieser als nicht mehr zeitgemäß empfundenen Methoden. Der Ansatz der aktuellen Forschungen geht in die Bestimmung der Belegereife anhand der Luftfeuchtigkeit, anstatt der Bestimmung des Wassergehalts im Baustoff gemäß Calciumcarbid-Methode in CM-%. Auch bei Wand- und Decken-Fertigteilen gibt es ähnliche Problemstellungen.

Aus dem Stand der Technik sind bereits unterschiedliche Ausführungsformen von Feuchtemessgeräten bekannt. So ist beispielsweise im Dokument US 7,231,815 B2 eine elektronische Messvorrichtung zur Bestimmung der relativen Feuchte von Beton beschrieben, wobei mit diesem Gerät beispielsweise auch die relative Luftfeuchtigkeit im Estrich angezeigt werden kann. Nachteilig bei dieser Ausführung ist zumindest, dass hier nachträglich ein Bohrloch in den Estrich gebohrt werden muss, in welches Bohrloch die elektronische Messvorrichtung dann eingesetzt wird. Während des Bohrvorgangs wird allerdings unter Umständen die Feuchte des angrenzenden Estrichs verfälscht, da beim mechanischen Bohren Wärme frei wird und die Feuchte des angrenzenden Materials durch das Bohrloch hindurch nach außen an die Umgebung abgegeben wird. Die Luftfeuchtigkeit wird mittels eines elektronischen Sensors, der im Inneren der Messvorrichtung eingebaut ist, bestimmt. Eine Anzeige am Kopf der elektronischen Messvorrichtung zeigt den eigentlichen Feuchtemesswert, ein Benutzer erhält dabei jedoch keinen direkten Hinweis auf die Belegereife, ob also der Estrich bereits genügend trocken ist, um einen Bodenbelag darauf zu verlegen. Der Benutzer muss daher wissen, bei welcher relativen Luftfeuchtigkeit im Estrich von einer Belegereife ausgegangen werden kann. Aufgrund der nachträglichen Einbringung und der aufwendigen Bauart ist eine solche elektronische Messvorrichtung jedoch auch in der Erzeugung viel teurer, was deren einmalige Verwendung in großer Anzahl bzw. an zahlreichen Messstellen pro Baustelle damit ausschließt.

Aus der DE 10 2012 208 050 A1 ist ein Feuchtemessgerät für Fußböden bekannt, welches auch zur Messung der relativen Luftfeuchtigkeit im Estrich geeignet ist. Auch bei dieser Ausführung ist ein elektronischer Luftfeuchtesensor vorgesehen, der im Estrichbett mit einbetoniert wird. Dieses Feuchtemessgerät besitzt keine direkte Anzeige, weshalb Messwerte drahtlos nach außen an ein Auswerte- bzw. Anzeigegerät übertragen werden müssen. Auch für diese Ausführung sind dieselben vorgenannten Nachteile anzuführen, weshalb der Einsatz dieses Feuchtemessgeräts für die einmalige Verwendung in großer Anzahl bzw. an zahlreichen Messstellen pro Baustelle ebenfalls ausgeschlossen ist.

Außerdem ist aus dem Dokument US 5,730,024 A ein Feuchtetestgerät bekannt, das mit seinem Gehäuse in einem strukturierten, feuchten Testmaterial wie beispielsweise in feuchtem Beton eingegossen wird. Innerhalb des dampfdurchlässigen Gehäuses befindet sich ein hygroskopisches Material, in welchem sich zwei voneinander beabstandete Elektroden befinden. Von den Elektroden wird dabei der elektrische Widerstand in Abhängigkeit von der Feuchte im Gehäuseinnenraum gemessen. Dafür werden Signalkabel von den beiden Elektroden aus dem Feuchtetestgerät sowie durch den feuchten Beton hindurch nach außen geführt, wobei sich das eigentliche Widerstandsmessgerät und die Messwertanzeige außerhalb des zu untersuchenden Testmaterials befinden. Nachteilig ist auch hier, dass das Feuchtetestgerät vergleichsweise aufwendig in seiner Herstellung ist. Weshalb auch für diese Ausführung dieselben vorgenannten Nachteile anzuführen sind, sowie der Einsatz dieses Feuchtetestgeräts für die einmalige Verwendung in großer Anzahl ebenfalls auch aus Kostengründen ausgeschlossen ist.

Außerdem ist bei den vorgenannten Ausführungen von Nachteil, dass bei der einmaligen Verwendung der vorgenannten Feuchtemessgeräte elektronische Bauteile im Bauwerk, also im Estrich bzw. in Gebäudewänden oder -decken zurückbleiben, was nicht nur aus wirtschaftlicher Sicht, sondern auch aus ökologischen Überlegungen kritisch ist.

Eine Feuchteindikatorvorrichtung zur hygrometrischen Bestimmung der Restfeuchte eines Baustoffs (Erdboden) ist bekannt aus US 3 117 442 A1.

Es ist somit Aufgabe der vorliegenden Erfindung eine Feuchteindikatorvorrichtung zur hygrometrischen Bestimmung der Materialfeuchte eines Baustoffs bereitzustellen, die die geschilderten Nachteile des Standes der Technik vermeidet, welche möglichst robust und kostengünstig herzustellen ist und welche auf rein mechanischem Wege eine Anzeige der Belegereife des entsprechenden Baustoffs bzw. Gebäudeteils ermöglicht.

Diese Aufgabe wird von einer Feuchteindikatorvorrichtung gemäß dem Oberbegriff des Anspruches 1 mit den Merkmalen des kennzeichnenden Teiles des Anspruchs 1 gelöst. Vorteilhafte Ausgestaltungen und Fortbildungen der Erfindung sind in den Unteransprüchen und der Beschreibung dargelegt.

Erfindungsgemäß ist bei einer Feuchteindikatorvorrichtung zur hygrometrischen Bestimmung der Restfeuchte eines Baustoffs, umfassend ein Gehäuse mit zumindest einem Gehäuseinnenraum, der über zumindest eine mit einer wasserdampfdurchlässigen Membran bedeckten Diffusionsöffnung mit der Gehäuseumgebung kommuniziert, wobei das Gehäuse ein Anzeigefeld aufweist, im Gehäuseinnenraum zumindest ein hygrisches Längenänderungselement angeordnet, welches hygrische Längenänderungselement abhängig von der im Gehäuseinnenraum herrschenden relativen Luftfeuchte jeweils unterschiedliche Längen einnimmt und mit einem mechanischen Auslösemechanismus in Wirkverbindung steht, wobei bei Unterschreiten einer voreinstellbaren Schwellenwerts der relativen Luftfeuchte der mechanische Auslösemechanismus in Abhängigkeit der eingenommenen Länge des Längenänderungselements im Anzeigefeld ein Anzeigemittel zur Anzeige der Schwellwertunterschreitung der voreingestellten relativen Luftfeuchte aktiviert.

Unter einem hygrischen Längenänderungselement wird im Weiteren ein Element verstanden, das aus zumindest einem hygrischen Material hergestellt ist bzw. zumindest ein hygrisches Material umfasst, das vorteilhaft die folgenden Eigenschaften aufweist:
- möglichst großer Ausdehnungskoeffizient des hygrischen Materials bei Feuchtigkeitszunahme zumindest in einer Längenrichtung des Materials;
- möglichst reversibler, bevorzugt auch linearer Zusammenhang zwischen der Ausdehnung bzw. Längenänderung des hygrischen Materials und der relativen Luftfeuchte;
- möglichst großer Elastizitäts-Modul (E-Modul) des Materials, um eine möglichst kraftunabhängige Längenänderung des hygrischen Materials zu gewährleisten;
- möglichst kleine Wärmedehnung des verwendeten hygrischen Materials zumindest im für Feuchteuntersuchungen bei Baustoffen relevanten Temperaturbereich von 0°C bis 30°C;
- ausreichende Festigkeit des hygrischen Materials.

Besonders vorteilhaft dient die erfindungsgemäße Feuchteindikatorvorrichtung zur Bestimmung und optischen Anzeige des Feuchtegehalts von Baustoffen. Die Feuchteindikatorvorrichtung wird beispielsweise während der Herstellung eines Fließestrichs in das nasse, frisch gegossene Estrichbett so eingebracht, dass das Anzeigefeld des Gehäuses sichtbar bleibt. Als Anzeigefeld des Gehäuses kann beispielsweise ein transparentes oder transluzentes Anzeigefenster dienen, wobei die Erfindung nicht auf Anzeigefenster als Anzeigefeld beschränkt ist. Im Falle einer elektronischen oder elektromechanischen Anzeige können auch ein oder mehrere elektronische Anzeigefelder am Gehäuse angeordnet sein.

Das Aushärten des Estrichs geht mit einer Abnahme der Feuchtigkeit des Estrichmaterials einher, welche Feuchte wiederum über eine Sorptionsisotherme mit der relativen Luftfeuchtigkeit korreliert. Das Gehäuse der Feuchteindikatorvorrichtung weist dazu eine oder mehrere Diffusionsöffnungen auf, die jeweils mit einer wasserdampfdurchlässigen Membran bedeckt sind. Somit steht der zumindest eine Gehäuseinnenraum mittels der Diffusionsöffnungen mit der Gehäuseumgebung in Verbindung und die relative Luftfeuchte im Gehäuseinnenraum korreliert mit dem Feuchtegehalt des Estrichbetts.

Das hygrische Längenelement, das im Gehäuseinnenraum angeordnet ist, löst bei Erreichen einer bestimmten Luftfeuchtigkeit, die als Schwellenwert vorbestimmbar ist und beispielsweise jener Baustofffeuchte des Estrichbetts entspricht, ab der ein Belegen des Estrichs mit einem Fußbodenmaterial ohne Bedenken möglich ist, einen mechanisch wirkenden Auslösemechanismus aus. Bei Unterschreiten des voreinstellbaren Schwellenwerts der relativen Luftfeuchte aktiviert der mechanische Auslösemechanismus in Abhängigkeit der eingenommenen Länge des Längenänderungselements im Anzeigefeld ein Anzeigemittel. Das Anzeigemittel dient zur Anzeige der Schwellwertunterschreitung der voreingestellten relativen Luftfeuchte und zeigt beispielhaft die Belegereife des Estrichs an.

Zusätzlich kann eine wasserdichte, aber dampfdurchlässige Schutzhülse, die ein Eindringen von Wasser und Zementschlamm in den Reaktionsmechanismus verhindert, an der Gehäuseaußenseite der Indikatorvorrichtung angeordnet sein. Bei Einsatz einer solchen Schutzhülse ist aber trotzdem sichergestellt, dass der Reaktor die relative Luftfeuchtigkeit im Estrich messen kann.

Selbstredend kann die erfindungsgemäße Feuchteindikatorvorrichtung neben der Bestimmung der Belegereife von Estrich auch zur hygrometrischen Bestimmung diverser Baustoffe bzw. Baumaterialien sowie zur Bestimmung der Restfeuchte des Mauerwerks von Gebäuden beispielsweise bei Mauertrockenlegungs- bzw. Sanierungsaufgaben, bei denen jeweils die Bestimmung und Anzeige einer definierten Restfeuchte eines Baustoffs bzw. Gebäudeteils von Interesse ist, eingesetzt werden.

Vorteilhaft kann die Feuchteindikatorvorrichtung aufgrund des mechanischen Auslösemechanismus kostengünstig hergestellt und besonders einfach vor Ort eingesetzt werden. Die Handhabung der Feuchteindikatorvorrichtung ist derart einfach, dass dazu kein Fachwissen erforderlich ist. Somit lässt sich bei Einsatz von entsprechenden Feuchteindikatorvorrichtungen direkt auf einer Baustelle von jedermann sehr einfach die Belegereife von Estrichen oder Gebäudeteilen feststellen.

Weiters ist von Vorteil, dass die Feuchteindikatorvorrichtung in eingebauter Lage in einem Baustoff bzw. in einem Gebäudeteil verbleiben kann und nicht entfernt werden muss, da aufgrund der rein mechanischen Bauweise des Auslösemechanismus auch keine problematischen oder schädlichen elektrischen Bauteile bzw. Batterien zum Betrieb der Indikatorvorrichtung vorhanden sind. Somit können Feuchteindikatorvorrichtungen gemäß der Erfindung, nachdem das Anzeigemittel zur Anzeige der Belegereife aktiviert wurde, einfach vom entsprechenden Boden- oder Wandbelagsmaterial, beispielsweise keramische Fliesen oder Kacheln, mit überdeckt werden, ohne dass davor weitere Sanierungsmaßnahmen erforderlich wären.

Vorteilhaft kann bei einer Indikatorvorrichtung gemäß der Erfindung der mechanische Auslösemechanismus zumindest ein beweglich gelagertes Übertragungselement sowie zumindest ein beweglich gelagertes Auslöseelement umfassen, wobei das zumindest eine Übertragungselement sowie das zumindest eine Auslöseelement miteinander in Wirkverbindung stehen, sowie das Übertragungselement mit dem hygrischen Längenänderungselement bewegungsmäßig gekoppelt ist und dabei Änderungen der jeweils eingenommenen Länge des Längenänderungselements an das Auslöseelement überträgt, wobei das Auslöseelement bei Schwellwertunterschreitung der voreingestellten relativen Luftfeuchte das Anzeigemittel auslöst.

Je nach Ausführung können im mechanischen Auslösemechanismus ein Übertragungselement, das mit dem hygrischen Längenänderungselement gekoppelt ist, und ein Auslöseelement, das auf das Anzeigemittel wirkt bzw. dieses auslöst, auch gelenkig miteinander verbunden sein. Alternativ dazu können das Übertragungselement und das Auslöseelement auch einstückig miteinander verbunden sein.

Weiters ist es im Rahmen der Erfindung zur Sicherstellung einer möglichst störungsfreien Anzeige der Schwellwertunterschreitung der vordefinierten relativen Luftfeuchte vorgesehen, gegebenenfalls einen oder mehrere Bauteile des mechanischen Auslösemechanismus mit geeigneten Federelementen vorzuspannen. Dazu ist prinzipiell der Einsatz von Zugfederelementen, Druckfederelementen und/oder von Biegefederelementen denkbar. Ebenso ist es im Rahmen der Erfindung vorgesehen, zumindest einzelne Bauteile des mechanischen Auslösemechanismus mit Druck zu beaufschlagen und für den Fall der Schwellwertunterschreitung der voreingestellten relativen Luftfeuchte das Auslösen des Anzeigemittels zumindest teilweise durch Druckausgleich zu bewerkstelligen. Die Druckbeaufschlagung kann dabei beispielsweise mittels Druckluft oder eines komprimierten Sicherheitsgases erfolgen.

Zweckmäßig kann bei einer erfindungsgemäßen Indikatorvorrichtung der mechanische Auslösemechanismus ein Sicherungselement umfassen, welches Sicherungselement von einer verriegelten Position in eine entsicherte Position des Auslösemechanismus bewegbar ist, wobei in der verriegelten Position der Auslösemechanismus auch bei einer Luftfeuchte unterhalb des Schwellwerts der voreingestellten relativen Luftfeuchte deaktiviert ist. Vorteilhaft kann somit die Indikatorvorrichtung bei einer definierten relativen Luftfeuchte kalibriert und anschließend der Auslösemechanismus mittels Sicherungselement verriegelt bzw. deaktiviert werden. Woraufhin die Indikatorvorrichtung auch bei einer niedrigen Luftfeuchte gelagert werden kann, welche niedrige Luftfeuchte andernfalls bei entsichertem Auslösemechanismus zu einem Auslösen des Anzeigemittels führen würde.

In einer bevorzugten Ausführungsform der Erfindung kann bei einer Indikatorvorrichtung das zumindest eine Übertragungselement an einer in Längsachsenrichtung des hygrischen Längenänderungselements angeordneten freien Endfläche mit diesem bewegungsmäßig gekoppelt sein, wobei das hygrische Längenänderungselement an seiner in Längsachsenrichtung entgegengesetzt gelegenen freien Endfläche am Gehäuse, vorzugsweise mit einem Festlager, gelagert ist. In dieser vorteilhaften Ausgestaltung der Erfindung wird das hygrische Längenänderungselement so eingesetzt, dass es in seiner Längsachsenrichtung an zwei einander gegenüberliegenden Endflächen des Längenänderungselements gelagert ist. Die erste Endfläche des hygrischen Längenänderungselements ist am Gehäuse gelagert und vorzugsweise als Festlager ausgeführt. Die zweite, der ersten Endfläche gegenüberliegende Endfläche des hygrischen Längenänderungselements dient als Lagerpunkt des zumindest einen Übertragungselements, das mit dem Längenänderungselement bewegungsmäßig gekoppelt ist. Somit werden Längenänderungen des hygrischen Längenänderungselements abhängig von der jeweils herrschenden relativen Luftfeuchte im Gehäuseinnenraum in seiner Längsachsenrichtung an das Übertragungselement weiter übertragen.

Bei der Gestaltung des Übertragungselements ist im Rahmen der Erfindung vorgesehen, dass das Übertragungselement Längenänderungen des hygrischen Längenänderungselements weiter verstärkt, wodurch die Bewegungsamplitude des hygrischen Längenänderungselements vergrößert wird.

Besonders vorteilhaft ist in einer weiteren erfindungsgemäßen Ausführungsvariante der Indikatorvorrichtung zum Einstellen des Schwellwerts der bei einer definierten relativen Luftfeuchte eingenommenen Länge des Längenänderungselements die Lagerposition des hygrischen Längenänderungselements im Gehäuseinnenraum mittels eines Justiermechanismus, vorzugsweise mit einer Justierschraube, justierbar. Mit Hilfe eines Justiermechanismus ist somit vorteilhaft ein Feinabgleich der Länge des hygrischen Längenänderungselements bzw. dessen Lagerposition möglich, wodurch gegebenenfalls Fertigungsungenauigkeiten der Feuchteindikatorvorrichtung ausgeglichen werden können.

Zweckmäßig kann bei einer Indikatorvorrichtung gemäß der Erfindung das Übertragungselement ein Haltemittel umfassen, welches Haltemittel abhängig von der jeweils eingenommenen Länge des Längenänderungselements bei einer Luftfeuchte oberhalb der voreingestellten relativen Luftfeuchte entlang eines bahnförmigen Gleitabschnitts des Auslöseelements gleitet, wobei das Haltemittel bei Unterschreiten des Schwellwerts der voreingestellten relativen Luftfeuchte den bahnförmigen Gleitabschnitt des Auslöseelements verlässt, wodurch die Stützfunktion des Haltemittels für das Auslöseelement endet und das Auslöseelement aus seiner Ruhelage auslöst bzw. seine Ruhelage verlässt. In dieser Ausführung gleitet ein Haltemittel des Übertragungselements, beispielsweise ein Haltebügel, bei Längenänderungen des hygrischen Längenänderungselements am Auslöseelement entlang. Sobald der Schwellwert der voreingestellten relativen Luftfeuchte unterschritten wird, verlässt das Haltemittel bzw. der Haltebügel des Übertragungselements den Gleitabschnitt des Auslöseelements und vom mechanischen Auslösemechanismus wird ein Anzeigemittel zur Anzeige der Schwellwertunterschreitung der relativen Luftfeuchte im Gehäuseinnenraum der Feuchteindikatorvorrichtung aktiviert.

In einer weiteren bevorzugten Ausführungsvariante kann bei einer erfindungsgemäßen Indikatorvorrichtung das Sicherungselement auf ein im Wesentlichen bügelförmiges Auslöseelement wirken und eine Stützfläche umfassen, welche Stützfläche abhängig von der jeweils eingenommenen Länge des Längenänderungselements bei einer ansteigenden Luftfeuchte oberhalb der voreingestellten relativen Luftfeuchte entlang des bahnförmigen Gleitabschnitts des Auslöseelements gleitet, bis die Stützfläche des Sicherungselements in eine Entsicherungsausnehmung des Auslöseelements eingreift und dabei das Haltemittel anstelle der Stützfläche die Stützfunktion des Auslöseelements übernimmt. Daraufhin wird das Auslöseelement vom Haltemittel des Übertragungselements in seiner Ruhelage gehalten. Erst bei Unterschreiten des Schwellwerts der voreingestellten relativen Luftfeuchte verlässt auch das Haltemittel den Gleitabschnitt des Auslösemittels, woraufhin dieses aus seiner Ruhelage auslöst bzw. diese Ruhelage verlässt. Daraufhin wird der mit dem Auslöseelement gekoppelte Auslösemechanismus zur Anzeige der Schwellwertunterschreitung der voreingestellten relativen Luftfeuchte aktiviert.

Wie eingangs bereits festgehalten, können einzelne oder auch sämtliche Bauteile des mechanischen Auslösemechanismus erforderlich durch zusätzliche Federelemente vorgespannt sein.

In einer alternativen Ausführungsvariante kann bei einer erfindungsgemäßen Indikatorvorrichtung das Sicherungselement eine zweistufige Auslösesicherung mit einem ersten Sicherungsmittel und einem zweiten Sicherungsmittel umfassen, welche Sicherungsmittel auf eine Bolzenhalterung eines im Wesentlichen bolzenförmigen Auslöseelements wirken, wobei in einer verriegelten Position des Auslösemechanismus das erste Sicherungsmittel in die Bolzenhalterung eingreift und in einer entsicherten Position des Auslösemechanismus abhängig von der jeweils eingenommenen Länge des Längenänderungselements bei einer Luftfeuchte oberhalb der voreingestellten relativen Luftfeuchte solange das zweite Sicherungsmittel in die Bolzenhalterung eingreift, bis bei Unterschreiten des Schwellwerts der voreingestellten relativen Luftfeuchte aufgrund der korrespondierenden eingenommenen Länge des Längenänderungselements das zweite Sicherungsmittel die Bolzenhalterung freigibt. Die Mechanik für die Entriegelung und Auslösung ist in diesem Fall ähnlich zu mechanischen Uhren bzw. Läutwerken, wobei beim Ausdehnen ein Schlagbolzen um einen Zahn des mechanischen Uhrwerks weiterspringt, aber erst beim darauffolgenden Kontrahieren dieser wirklich freigängig wird.

Ein bolzenförmiges Auslöseelement bietet den Vorteil, dass dieses zur Anzeige der Schwellwertunterschreitung der voreingestellten relativen Luftfeuchte direkt oder indirekt ein oder mehrere Anzeigemittel aktivieren kann oder aber bei Auslösen der Auslösesicherung das Gehäuse der Feuchteindikatorvorrichtung beispielsweise im Bereich des Anzeigefelds durchstößt und derart selbst ein Anzeigemittel bildet. Vorzugsweise ist dazu das bolzenförmige Auslöseelement mit einer Vorspannfeder oder einem sonst geeigneten Vorspannmittel, beispielsweise mittels Druckluft, vorgespannt.

Zweckmäßig kann bei einer Indikatorvorrichtung gemäß der Erfindung das hygrische Längenänderungselement ausgewählt sein aus einer Gruppe umfassend:
- Holz, vorzugsweise Buchenholz;
- Naturhaar menschlichen oder tierischen Ursprungs;
- Papier und/oder Karton;
- Kunststoff, vorzugsweise Polyamid;
- Absorberelement, befüllt mit Superabsorber;
- Absorberelement, befüllt mit einem hygrischen Mineralstoff oder hygrischen Mineralstoffgemenge, vorzugsweise mit Bentonit und/oder Montmorillonit.

Besonders vorteilhaft können im Rahmen der Erfindung je nach Anwendungsfall unterschiedliche hygrische Längenänderungselemente eingesetzt werden. Beispielsweise kann ein Holzelement, vorzugsweise aus Buchenholz, zur Bestimmung der Feuchteänderung dienen. Der Werkstoff Holz bietet dabei zahlreiche Vorteile, da Holz kostengünstig und leicht verfügbar sowie mechanisch stabil und porös ist. Aufgrund seines großen Dehnungskoeffizienten ist Holz bei gleichzeitig geringer Wärmedehnung daher sehr gut für den Feuchtigkeitstransport geeignet.

Haare werden üblicherweise zur Feuchtebestimmung in Hygrometern eingesetzt, da sie vorteilhaft eine große Oberfläche besitzen und ebenfalls sehr gut für den Feuchtigkeitstransport geeignet sind. Außerdem können Haare beispielsweise über Umlenkrollen geführt werden, um eine Koppelung mit dem Auslösemechanismus zu erzielen.

Ebenso können im Rahmen der Erfindung Kunststoffe, vorzugsweise Polyamid, als hygrisches Längenänderungselement, eingesetzt werden. Aufgrund ihrer vergleichsweise geschlossenen Oberfläche haben Kunststoffe im Vergleich zu offenporigem Holz zwar eine langsamere Feuchtigkeitsaufnahme bzw. -abgabe, also eine geringere Diffusionsgeschwindigkeit als Holz, allerdings bieten Kunststoffe den Vorteil einer hohen Reproduzierbarkeit der Längenänderung in Abhängigkeit von der relativen Feuchte. Außerdem weisen Kunststoffe gute mechanische Eigenschaften auf und sind robust.

Auch der Einsatz von Mineralstoffen wie beispielsweise Bentonit und/oder Montmorillonit als hygrisches Längenänderungselement ist im Rahmen der Erfindung vorgesehen. Ebenso können sogenannte Superabsorber (englisch: Superabsorbent Polymers, SAP) als hygrisches Längenänderungselement verwendet werden. Superabsorber werden Kunststoffe genannt, die in der Lage sind, ein Vielfaches ihres Eigengewichts an polaren Flüssigkeiten aufzusaugen. Dies sind vor allem Wasser bzw. wässrige Lösungen. Bei der Aufnahme der Flüssigkeit quillt der Superabsorber auf und bildet ein Hydrogel. Die Summe aus dem Volumen der Flüssigkeit und dem Volumen des trockenen Superabsorbers bleibt dabei gleich. Superabsorber kommen üblicherweise als grobkörniges Pulver mit Partikelgrößen von 0,1 mm bis 1,0 mm zum Einsatz. Aufgrund ihrer pulverförmigen Struktur werden Superabsorber daher in dampfdurchlässige Gebinde, beispielsweise in Käfige, befüllt und als Absorberelemente eingesetzt. Ebenso werden Mineralstoffe als Absorberelemente gepackt.

Beispielsweise kann dazu auch ein Abschnitt des Gehäuseinnenraums als Absorberelement dienen, indem dieser Innenraumabschnitt mit Mineralstoffen oder mit Superabsorber befüllt ist und darüber ein in Längsachsenrichtung des Gehäuses verschiebbarer Kolben gelagert ist. Mittels einer Hilfsfeder wird erreicht, dass bei geringerer Luftfeuchte, also beim zumindest teilweisen Trocknen der Mineralstoffe bzw. Superabsorber-Pulver wieder eine geordnete Komprimierung des Absorberelements zu gewährleisten.

Falls beim Einsatz von Mineralstoffen wie Bentonit diese eine zu hohe Viskosität bzw. Scherfestigkeit aufweisen, um selbst als Hydraulikflüssigkeit zu dienen, so kann erforderlichenfalls ein hydraulischer Umsetzer eingebaut werden, um je nach herrschender relativer Feuchte im Gehäuseinnenraum eine ausreichende bzw. definierte Feuchtelängenänderung des Absorberelements zu gewährleisten.

Vorteilhaft stehen somit je nach Anwendungsfall für unterschiedliche Estricharten bzw. Zusatzstoffe von Estrichen, sowie für unterschiedliche Baustoffe unterschiedliche Indikatoren als hygrische Längenänderungselemente zur Verfügung. Damit kann auch je nach Aufgabenstellung eine Belegereife des zu untersuchenden Baustoffes bei unterschiedlichen relativen Luftfeuchten angezeigt werden.

Besonders vorteilhaft kann bei einer erfindungsgemäßen Indikatorvorrichtung das Anzeigemittel zur Anzeige der Schwellwertunterschreitung der voreingestellten relativen Luftfeuchte ausgewählt sein aus einer Gruppe umfassend:
- mechanisches Anzeigemittel;
- Farbanzeigemittel;
- elektromechanisches Anzeigemittel;
- elektronisches Anzeigemittel.

Als mechanische Anzeigemittel kommen beispielsweise Skalenanzeigen zum Einsatz. Als Farbanzeigemittel ist beispielsweise der Einsatz von Farbkapseln vorgesehen, die bei Unterschreiten der Schwellwertfeuchte aufgesprengt werden und eine irreversible Farbanzeige bilden. Ebenso können elektrische und/oder elektromechanische Anzeigemittel, beispielsweise Schauzeichen oder Lichtsignale bzw. eine Flüssigkristallanzeige dienen. Die Messung der Luftfeuchtigkeit kann dabei über an sich bekannte Sensoren erfolgen und wird durch eine Analogschaltung oder einen Mikroprozessor erfasst. Sowohl die Schwellwertschaltung als auch die Entriegelung sind in der Schaltung/Software implementiert. Die Energieversorgung erfolgt beispielhaft mittels unschädlicher Batterien oder Photovoltaikelemente.

Um die Anzeige der Belegereife auch unter widrigen Bedingungen, also beispielsweise bei verschmutztem oder zerkratztem Anzeigefenster oder bei schlechter Beleuchtung gut zu erkennen, ist daher denkbar, den oberen Gehäuseabschluss der Feuchteindikatorvorrichtung transparent auszuführen und aus dem Estrich, Beton oder Baustoff im Testeinsatz herausragen zu lassen bzw. derart in diesen einzubauen, dass dieser bündig mit der Baustoffoberfläche abschließt. Durch entsprechende Handhabung muss gewährleistet werden, dass der Großteil der transparenten Anzeigefläche sauber bleibt bzw. gesäubert wird.

Unterhalb des transparenten Anzeigefensters werden bei Unterschreitung der Schwellwertfeuchte beispielsweise farblich gekennzeichnete Bauteile sichtbar oder verdeckt, etwa durch das Abtauchen eines farbigen Stifts oder durch Verdrehen von farbigen Lamellen, die von andersfarbigen verdeckt werden. Ebenso denkbar ist eine Farbkapsel, die durch ein bewegliches Auslöseelement zerstört wird und das unterhalb der transparenten Schicht befindliche saugfähige Material einfärbt.

Ebenso kann das Gehäuse der Feuchteindikatorvorrichtung undurchsichtig sein und wird nahezu bündig mit der Betonoberfläche eingegossen. Das Erreichen des Schwellwerts wird in diesem Fall beispielsweise dadurch angezeigt, dass ein Bauteil aus der Indikatorvorrichtung herausdringt, beispielsweise ein Auslöseschlagbolzen, der das Gehäuse und die anhaftende Beton-, Estrich- oder Baustoffschicht durchdringt und so unmittelbar sichtbar wird. Alternativ könnte auch eine kleine Menge an unschädlichem Farbstoff freigesetzt werden, der sich rund um den Messpunkt auf der Baustoffoberfläche verteilt. Außerdem ist im Rahmen der Erfindung vorgesehen, dass im Innenraum des Gehäuses bei Unterschreiten der Schwellwertfeuchte vom Auslösemechanismus beispielsweise eine chemische Reaktion in Gang gesetzt wird, die zugleich Druck und Wärme erzeugt. Dadurch wird der obere Gehäuseabschnitt, beispielsweise ein Gehäuseabschluss aus Kunststoff, plastisch und aufgrund des Überdrucks im Innenraum zu einem ballonförmigen Gebilde aufgeblasen, das auch anhaftenden Beton abplatzen lässt.

Erforderlichenfalls verhindert ein Gegengewicht, das an der Feuchteindikatorvorrichtung befestigt ist, beim Eintauchen der Feuchteindikatorvorrichtung in den feuchten Estrich oder Baustoff ein unerwünschtes Aufschwimmen.

Weitere Einzelheiten, Merkmale und Vorteile der Erfindung ergeben sich aus der nachfolgenden Erläuterung von in den Zeichnungen schematisch dargestellten Ausführungsbeispielen. In den Zeichnungen zeigen:
- **Fig. 1** in einer Längsschnittansicht von vorne eine erste erfindungsgemäße Ausführung einer Feuchteindikatorvorrichtung;
- **Fig. 2** in einer Schnittansicht von vorne die in Fig. 1 veranschaulichte Feuchteindikatorvorrichtung in Einbaulage in einem Baustoff;
- **Fig. 3A** bis **Fig. 9B** in einer Abfolge dieselbe erfindungsgemäße Feuchteindikatorvorrichtung bei unterschiedlichen relativen Luftfeuchten, wobei die Abbildungen Fig. 3A bis Fig. 9A die Indikatorvorrichtung jeweils im Schnitt von vorne und die Abbildungen Fig. 3B bis Fig. 9B dieselbe Indikatorvorrichtung jeweils im Schnitt von der Seite zeigen; und

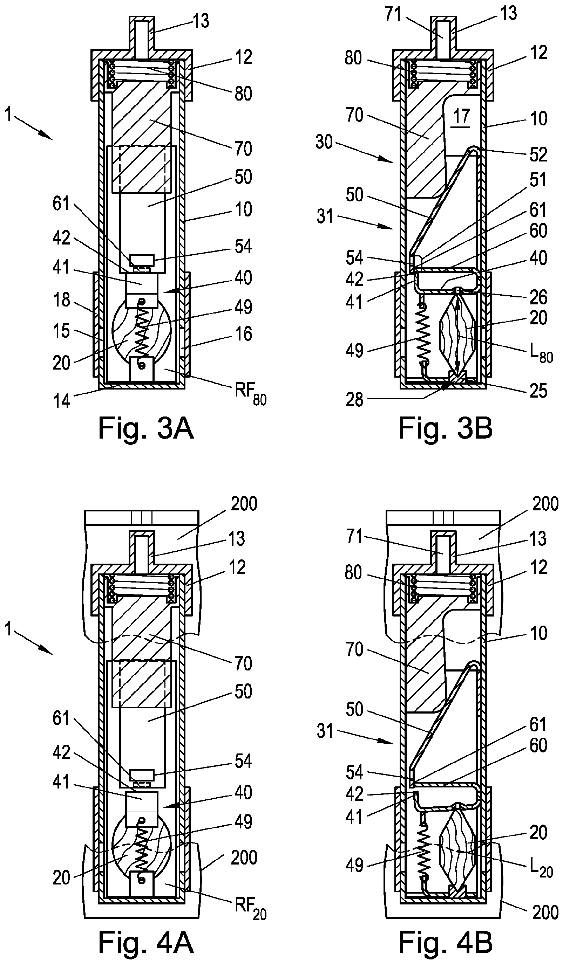

- **Fig. 3A** und **Fig. 3B** die Feuchteindikatorvorrichtung jeweils beispielhaft bei 80% relativer Luftfeuchte als vorgegebener Schwellwertfeuchte zum Justieren des Auslösemechanismus zeigen;
- **Fig. 4A** und **Fig. 4B** die Feuchteindikatorvorrichtung während der Lagerung mit verriegeltem Auslösemechanismus jeweils bei 20% relativer Luftfeuchte zeigen;

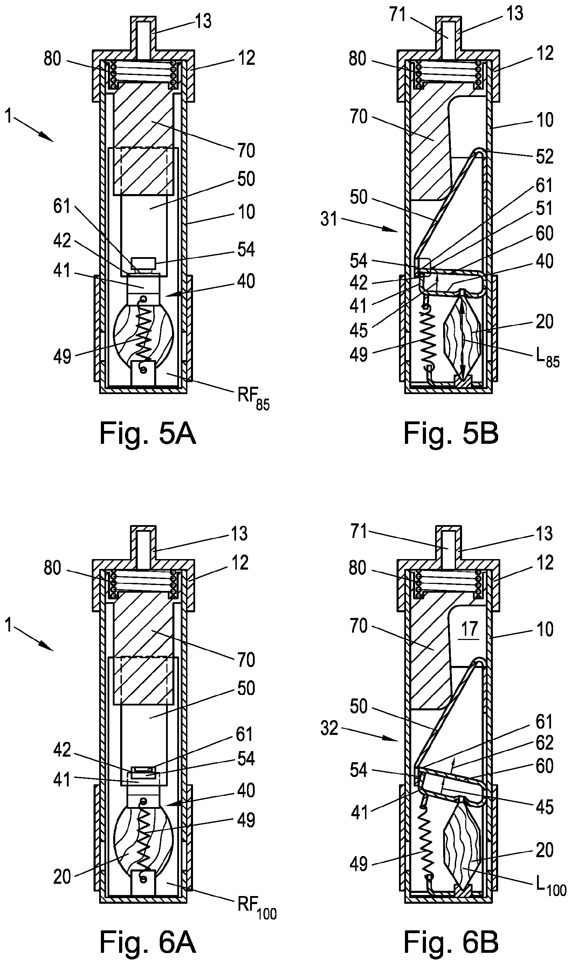

- **Fig. 5A** und **Fig. 5B** die Feuchteindikatorvorrichtung im Einsatz mit verriegeltem Auslösemechanismus jeweils bei 85% relativer Luftfeuchte zeigen;
- **Fig. 6A** und **Fig. 6B** die Feuchteindikatorvorrichtung im Einsatz mit entsichertem Auslösemechanismus im feuchten Baustoff jeweils bei 100% relativer Luftfeuchte zeigen;

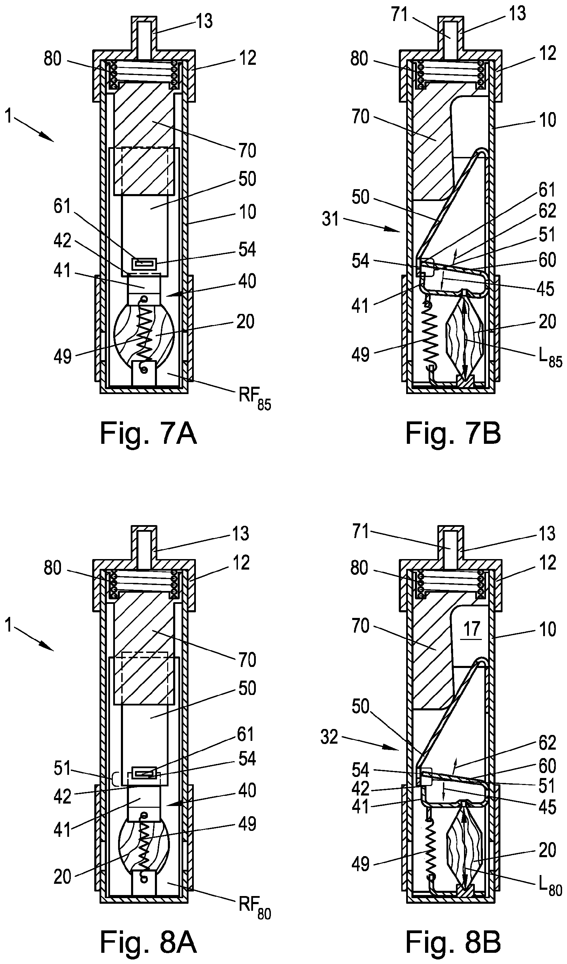

- **Fig. 7A** und **Fig. 7B** die Feuchteindikatorvorrichtung im Einsatz mit entsichertem Auslösemechanismus im bereits teilweise getrockneten Baustoff jeweils bei 85% relativer Luftfeuchte zeigen;
- **Fig. 8A** und **Fig. 8B** die Feuchteindikatorvorrichtung im Einsatz mit entsichertem Auslösemechanismus im bereits weiter getrockneten Baustoff jeweils bei 80% relativer Luftfeuchte, also bei Schwellwertfeuchte, zeigen;

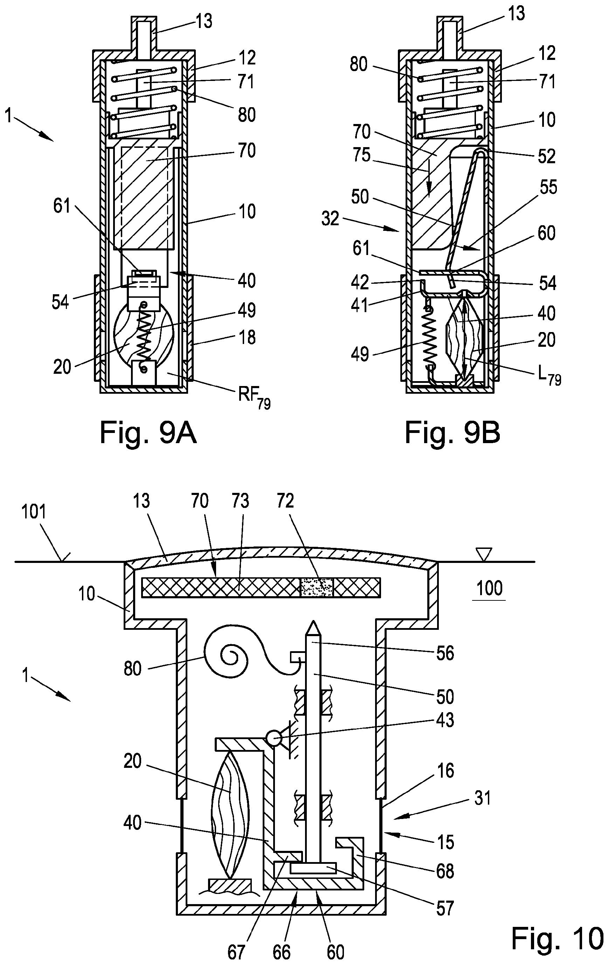

- **Fig. 9A** und **Fig. 9B** die Feuchteindikatorvorrichtung im Einsatz mit entsichertem Auslösemechanismus bei Unterschreiten der Schwellwertfeuchte bei 79% relativer Feuchte mit ausgelöster Anzeige darstellen;
- **Fig. 10** in einer Längsschnittansicht von vorne eine zweite erfindungsgemäße Ausführung einer Feuchteindikatorvorrichtung;

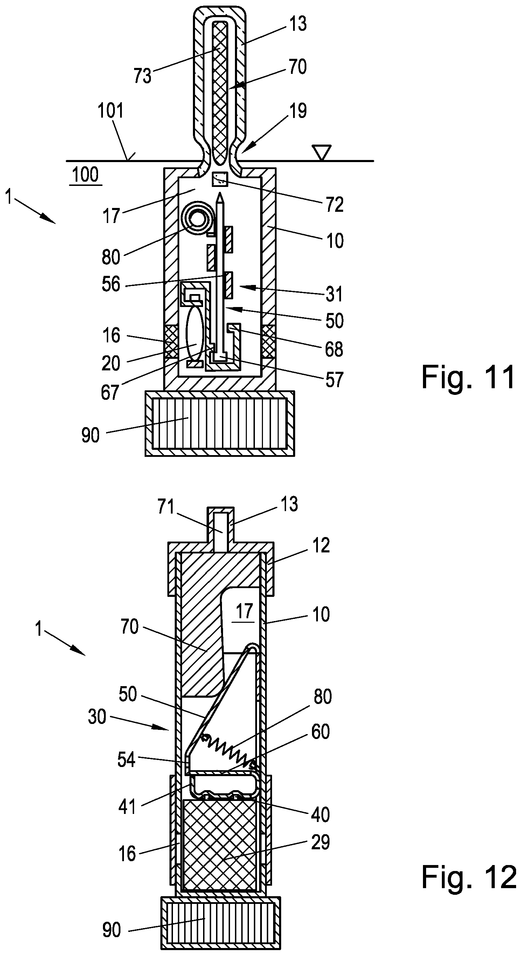

- **Fig. 11** in einer Längsschnittansicht von vorne eine dritte erfindungsgemäße Ausführung einer Feuchteindikatorvorrichtung;
- **Fig. 12** in einer Längsschnittansicht von vorne eine vierte erfindungsgemäße Ausführung einer Feuchteindikatorvorrichtung.

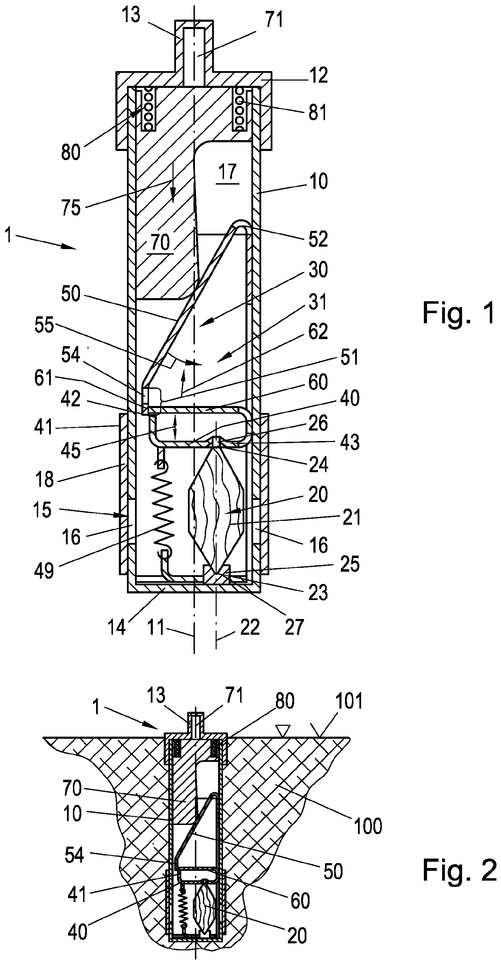

**Fig. 1** zeigt eine Feuchteindikatorvorrichtung 1 mit einem Gehäuse 10 bzw. Rohr 10. Das Gehäuse 10 weist eine Längsachse 11 sowie einen oberen Gehäuseabschluss 12 mit einem Anzeigefeld 13 bzw. Anzeigefenster 13 auf. Am gegenüberliegenden Ende ist das Gehäuse 10 mit einem Gehäuseboden 14 begrenzt, wobei hier zwei Diffusionsöffnungen 15, welche mit einer wasserdampfdurchlässigen Membran 16 bedeckt sind, eine kommunizierende Verbindung zwischen einem Innenraum 17 des Gehäuses 10 und der Gehäuseumgebung gewährleisten. Zum Schutz vor Beschädigung und Verunreinigung sind die Membrane 16 außenseitig des Gehäuses 10 noch mit einer dampfdurchlässigen Schutzhülse 18 geschützt.

Als hygrisches Längenänderungselement 20 ist hier ein Holzelement 21 beispielsweise aus Rotbuche vorgesehen, welches Holzelement 21 bei einer relativen Luftfeuchte etwa von 80% gezeigt ist. Das hier im Wesentlichen längliche Holzelement 21 weist eine Längsachse 22, eine erste freie Endfläche 23 sowie eine zweite freie Endfläche 24, welche der ersten freien Endfläche 23 gegenüberliegt, auf. Das Holzelement 21 ist an einem ersten Lagerpunkt 25 an der ersten freien Endfläche 23 sowie an einem zweiten Lagerpunkt 26 an der zweiten freien Endfläche 24 gelagert. Der erste Lagerpunkt 25 wird durch ein Festlager 27 gebildet, welches hier zusätzlich mit einem Justiermechanismus 28 bzw. mit einer Justierschraube 28 in seiner Position im Gehäuseinnenraum 17 feineinstellbar ist.

Ein Auslösemechanismus 30, der in der hier in Fig. 1 gezeigten Ausführung ein beweglich gelagertes Übertragungselement 40, ein bewegliches Auslöseelement 50 sowie ein Sicherungselement 60 umfasst und mittels Indikatorelement 70 auf ein mechanisches Anzeigemittel 71 wirkt, ist in einer verriegelten Position 31 des Auslösemechanismus 30 gezeigt. Übertragungselement 40, bewegliches Auslöseelement 50 sowie Sicherungselement 60 und Indikatorelement 70 stehen miteinander in Wirkverbindung, wobei das Übertragungselement 40 mit dem hygrischen Längenänderungselement 20 bzw. hier mit dem Holzelement 21 bewegungsmäßig gekoppelt ist. Längenänderungen des hygrischen Längenänderungselements 20 bzw. Holzelements 21 in Abhängigkeit von der im Gehäuseinnenraum 17 jeweils herrschenden Luftfeuchte werden dabei vom Übertragungselement 40 an das beweglich gelagerte Auslöseelement 50 übertragen. Das Auslöseelement 50 löst bei Schwellwertunterschreitung der voreingestellten relativen Luftfeuchte hier indirekt eine Anzeige aus, indem vom Auslöseelement 50 ein zwischengeschaltetes Indikatorelement das mechanische Anzeigemittel auslöst.

Am Übertragungselement 40 ist ein Haltemittel 41 bzw. Haltebügel 41 angeordnet, wobei ein freies Ende 42 des Haltemittels 41 an einem bahnförmigen Gleitabschnitt 51 des Auslöseelements 50 anliegt und das Haltemittel 41 weiters gegen das Sicherungselement 60 drückt. Eine Lagereinrichtung 43 dient zur beweglichen Lagerung des Übertragungselements 40, welches sich in Bewegungsrichtung 45, die als Doppelpfeil 45 dargestellt ist, bewegen lässt. Ein Rückstellmittel 49, das hier als Hilfszugfeder 49 ausgeführt ist, dient dabei zur Unterstützung der Rückstellung des Übertragungselements 40, sobald das Längenänderungselement 20 bei vergleichsweise geringer Luftfeuchte eine reduzierte Länge aufweist.

Das bewegliche Auslöseelement 50 weist hier einen bahnförmigen Gleitabschnitt 51 auf und ist mit einem Biegegelenk 52 elastisch vorgespannt. Anstelle des gezeigten Biegegelenks könnte das Auslöseelement auch mit einem Federgelenk oder mit einem externen Vorspannmittel vorgespannt sein. Entlang des bahnförmigen Gleitabschnitts 51 befindet sich eine Entsicherungsausnehmung 54 im Auslöseelement 50. Die bewegliche Lagerung des Auslöseelements 50 ist als Drehbewegung 55 durch einen Pfeil 55 veranschaulicht.

Weiters befindet sich ein Sicherungselement 60 mit einer Stützfläche 61 innerhalb des Auslösemechanismus 30. Eine Biegebewegung 62 des beweglich gelagerten Sicherungselements 60 ist durch Pfeil 62 dargestellt. Ein Indikatorelement 70, welches hier mittels eines Vorspannmittels 80 in Längsachsenrichtung 11 verschiebbar gegen das Auslöseelement 50 gedrückt wird, wirkt auf ein mechanisches Anzeigemittel 71, welches innerhalb des Anzeigefensters 13 angeordnet ist. Eine Bewegungsrichtung 75 des Indikatorelements 70, welche auch der Druckrichtung des Vorspannmittels 80 bzw. der Vorspannfeder 81 entspricht, ist hier durch den Pfeil 75 veranschaulicht.

**Fig. 2** zeigt die in Fig. 1 veranschaulichte Feuchteindikatorvorrichtung 1 in Einbaulage in einem Baustoff 100, beispielsweise in einem Estrich, mit einer Oberfläche 101 des Baustoffs 100.

**Fig. 3A** und **Fig. 3B** zeigen die Feuchteindikatorvorrichtung 1 jeweils beispielhaft bei 80% relativer Luftfeuchte RF₈₀, welche hier als Schwellwert für die Belegereife erzielt werden soll und bei der die Feuchteindikatorvorrichtung 1 auslösen soll. Somit wird der Auslösemechanismus 30 gemäß den Ansichten von Fig. 3A bzw. 3B eben bei der Schwellwertfeuchte RF₈₀ kalibriert. Dies wird beispielsweise in einer Klimakammer bei exakt 80% relativer Luftfeuchte RF₈₀ durchgeführt und somit gewährleistet, dass eine Länge L₈₀ des hygrischen Längenänderungselements 20 bzw. Holzelements 21 auch mit 80% relativer Luftfeuchte RF₈₀ korreliert. Dazu wird der Auslösemechanismus 30 so eingestellt, dass die Stützfläche 61 des Sicherungselements 60 am bahnförmigen Gleitabschnitt 51 des Auslöseelements 50 von der Entsicherungsausnehmung 54 etwas beabstandet ansteht. Der Auslösemechanismus 30 befindet sich somit in einer verriegelten Position 31, da das Auslöseelement 50 durch das Sicherungselement 60 in seiner Lage fixiert ist und nicht in Pfeilrichtung 55 drehbar bzw. schwenkbar bewegt werden kann. Das Übertragungselement 40 ist weiters so eingestellt, dass das freie Ende 42 seines Haltebügels 41 am Sicherungselement 60 anliegt oder sich zumindest in nächster Nähe zum Sicherungselement 60 befindet, ohne aber dieses in seiner Lage in Pfeilrichtung 62 zu bewegen.

**Fig. 4A** und **Fig. 4B** zeigen die Feuchteindikatorvorrichtung 1 während der Lagerung mit verriegeltem 31 Auslösemechanismus 30 jeweils bei 20% relativer Luftfeuchte. Dazu befindet sich die Feuchteindikatorvorrichtung 1 beispielsweise in einer Schutzverpackung 200. Die Länge L₂₀ des hygrischen Längenänderungselements 20 korreliert mit 20% relativer Luftfeuchte RF₂₀. Das Übertragungselement 40 ist in Pfeilrichtung 45 vom Sicherungselement 60 etwas beabstandet, wobei diese Verschwenkung 45 auch durch die Hilfszugfeder 49 unterstützt ist.

**Fig. 5A** und **Fig. 5B** zeigen die Feuchteindikatorvorrichtung 1 im Einsatz mit verriegeltem 31 Auslösemechanismus 30 jeweils bei 85% relativer Luftfeuchte RF₈₅. Die Länge L₈₅ des hygrischen Längenänderungselements 20 korreliert dabei mit 85% relativer Luftfeuchte RF₈₅. Wie in Fig. 5A verdeutlicht ist befindet sich hier die Stützfläche 61 des Sicherungselements 60 noch entlang des bahnförmigen Gleitabschnitts 51 des Auslöseelements 50 und verriegelt dieses.

**Fig. 6A** und **Fig. 6B** zeigen die Feuchteindikatorvorrichtung 1 im Einsatz mit entsichertem 32 Auslösemechanismus 30 im feuchten Baustoff 100, beispielsweise in feuchtem Estrich, jeweils bei 100% relativer Luftfeuchte RF₁₀₀ im Gehäuseinnenraum 17. Die Länge L₁₀₀ des hygrischen Längenänderungselements 20 korreliert dabei mit 100% relativer Luftfeuchte RF₁₀₀. Durch die maximale Länge L₁₀₀ des hygrischen Längenänderungselements 20 bzw. Holzelements 21 wird das Sicherungselement 60 in Pfeilrichtung 62 soweit nach oben gedrückt, dass die Stützfläche 61 entlang des Gleitabschnitts 51 am Auslöseelement 50 bis zur Entsicherungsausnehmung 54 bewegt wird und die Stützfläche 61 in die Entsicherungseinrichtung 54 eingreift. Dadurch übernimmt nun das Haltemittel 41 anstelle der Stützfläche 61 die Stützfunktion des Auslöseelements 50. Somit wird das Auslöseelement 50 nicht mehr gesichert und der Auslösemechanismus 30 ist nun in entsicherter Position 32. Übertragen wird die Längenänderung des Holzelements 21 zu seiner maximalen Länge L₁₀₀ durch das Übertragungselement 40, wobei der Haltebügel 41 des Übertragungselements 40 nun am bahnförmigen Gleitabschnitt 51 des Auslöseelements 50 anliegt. Der Haltebügel 41 sichert daher vorerst - anstelle des bereits entsicherten Sicherungselements 60 - weiterhin das Auslöseelement 50 in seiner Ruhelage und verhindert dadurch ein unerwünschtes vorzeitiges Auslösen der Indikatorvorrichtung 1.

**Fig. 7A** und **Fig. 7B** zeigen die Feuchteindikatorvorrichtung 1 im Einsatz mit entsichertem 32 Auslösemechanismus 30 im bereits teilweise getrockneten Baustoff 100 jeweils bei 85% relativer Luftfeuchte RF₈₅. Die Länge L₈₅ des hygrischen Längenänderungselements 20 korreliert mit 85% relativer Luftfeuchte RF₈₅.

**Fig. 8A** und **Fig. 8B** zeigen die Feuchteindikatorvorrichtung 1 im bereits weiter getrockneten Baustoff 100 jeweils bei 80% relativer Luftfeuchte als vorgegebener Schwellwertfeuchte, wobei die Länge L₈₀ des hygrischen Längenänderungselements 20 mit 80% relativer Luftfeuchte RF₈₀ korreliert. Der Haltebügel 41 sichert gerade noch mit seinem freien Ende 42 das Auslöseelement 50 in seiner Lage und verhindert dadurch ein unerwünschtes vorzeitiges Auslösen der Indikatorvorrichtung 1.

**Fig. 9A** und **Fig. 9B** stellen die Feuchteindikatorvorrichtung 1 im Einsatz mit entsichertem 32 Auslösemechanismus 30 bei Unterschreiten der Schwellwertfeuchte von 80% hier bei 79% relativer Feuchte RF₇₉ mit bereits ausgelöster Anzeige 71 dar. Wiederum korreliert die Länge L₇₉ des hygrischen Längenänderungselements 20 mit 79% relativer Luftfeuchte RF₇₉. Der Haltebügel 41 des Übertragungselements 40 liegt nun nicht mehr stützend am Auslöseelement 50 an, sondern hat mit seinem freien Ende 42 bereits den bahnförmigen Gleitabschnitt 51 des Auslöseelements 50 verlassen, wodurch das vorgespannte Auslöseelement 50 nicht mehr gesichert war und in Pfeilrichtung 55 aus seiner Ruhelage weggedreht bzw. wegeklappt ist. Somit war der Weg in Pfeilrichtung 75 für das vorgespannte 80 Indikatorelement 70 frei, der Federkraft der Vorspannfeder 81 auszuweichen und in Längsachsenrichtung 11 nach unten zu gleiten. Somit wurde vom hygrischen Längenänderungselement 20 zur Anzeige der Schwellwertunterschreitung bei Erreichen der voreingestellten relativen Luftfeuchte RF₈₀ im Anzeigefeld 30 mittels des verschobenen Indikatorelements 70 ein mechanisches Anzeigemittel 71 aktiviert und so besonders komfortabel die Belegereife des Baustoffs 100 signalisiert.

Der Vollständigkeit halber sei hier nochmals festgehalten, dass auch beliebige andere relative Luftfeuchtewerte, beispielsweise Werte zwischen 55% und 85% relativer Luftfeuchte, als Schwellwerte zur Festlegung der Belegereife der zu untersuchenden Baustoffe, dienen können.

**Fig. 10** betrifft eine zweite erfindungsgemäße Ausführung einer Feuchteindikatorvorrichtung 1, bei der Auslöseschlagbolzen 56 als Auslöseelement 50 dient. Eine Bolzenhalterung 57, die am Auslöseschlagbolzen 56 angeordnet ist, wirkt hier mit einer zweistufigen Auslösesicherung 66 zusammen, die ein erstes Sicherungsmittel 67 sowie ein zweites Sicherungsmittel 68 umfasst. In einer verriegelten Position 31 des Auslösemechanismus 30 greift das erste Sicherungsmittel 67 in die Bolzenhalterung 57 ein und in einer entsicherten Position 32 des Auslösemechanismus 30 greift das zweite Sicherungsmittel 68 solange in die Bolzenhalterung 57 ein, solange die Luftfeuchte oberhalb der voreingestellten relativen Luftfeuchte RF₈₀ ist. Bei Unterschreiten RF₇₉ des Schwellwerts RF₈₀ der voreingestellten relativen Luftfeuchte von beispielsweise 80% relativer Feuchte gibt das Längenänderungselement 20 aufgrund seiner korrespondierenden eingenommenen Länge L₇₉ mittels Übertragungselement 40 das zweite Sicherungsmittel 68 und damit die Bolzenhalterung 57 frei. Der Auslöseschlagbolzen 56 prallt daraufhin hier gegen ein Farbanzeigemittel 72 bzw. gegen eine Farbkapsel 72. Die dabei austretende Farbe gelangt zu einem saugfähigen Material 73, wodurch mittels Farbänderung die Belegereife des Baustoffs 100 signalisiert wird.

**Fig. 11** zeigt eine dritte erfindungsgemäße Ausführung einer Feuchteindikatorvorrichtung 1, bei der eine Sollbruchstelle 19 zum Entfernen des Anzeigefelds 13 bzw. des Anzeigefensters 13 vorgesehen ist. Dieses kann nach erfolgtem Auslösen der Indikatorvorrichtung 1, wobei auch hier die Belegereife des Baustoffs 100 mittels Farbanzeigemittel 72 signalisiert wird, einfach abgebrochen werden. Das restliche Gehäuse der Feuchteindikatorvorrichtung 1 verbleibt im Baustoff 100. Ein Gegengewicht 90 verhindert beim Eintauchen der Feuchteindikatorvorrichtung 1 in den feuchten Estrich oder Baustoff ein unerwünschtes Aufschwimmen.

**Fig. 12** zeigt eine vierte erfindungsgemäße Ausführung einer Feuchteindikatorvorrichtung 1, wobei hier als hygrisches Längenänderungselement 20 ein Absorberelement 29 dient, das mit Superabsorber befüllt ist. Ein Gegengewicht 90 dient zur Stabilisierung der Lage der Feuchteindikatorvorrichtung 1 in eingebauter Lage in einem Baustoff bzw. Estrich.

### LISTE DER BEZUGSZEICHEN

- 1: Feuchteindikatorvorrichtung
- 10: Gehäuse (Rohr)
- 11: Längsachse des Gehäuses
- 12: (oberer) Gehäuseabschluss
- 13: Anzeigefeld (bzw. Anzeigefenster)
- 14: (unterer) Gehäuseboden
- 15: Diffusionsöffnung
- 16: Membran
- 17: Innenraum des Gehäuses
- 18: Schutzhülse
- 19: Sollbruchstelle zum Entfernen des Anzeigefelds
- 20: Hygrisches Längenänderungselement
- 21: Holzelement
- 22: Längsachse des Längenänderungselements (bzw. Holzelements)
- 23: erste freie Endfläche des Längenänderungselements (bzw. Holzelements)
- 24: zweite freie Endfläche des Längenänderungselements (bzw. Holzelements)
- 25: erster Lagerpunkt des Längenänderungselements (bzw. Holzelements)
- 26: zweiter Lagerpunkt des Längenänderungselements (bzw. Holzelements)
- 27: Festlager
- 28: Justiermechanismus (bzw. Justierschraube)
- 29: Absorberelement
- 30: Auslösemechanismus
- 31: verriegelte Position des Auslösemechanismus
- 32: entsicherte Position des Auslösemechanismus
- 40: Übertragungselement
- 41: Haltemittel des Übertragungselements (bzw. Haltebügel)
- 42: freies Ende des Haltemittels
- 43: Lagereinrichtung für Übertragungselement
- 45: Bewegungsrichtung des Übertragungselements (Doppelpfeil)
- 49: Rückstellmittel (bzw. Hilfszugfeder)
- 50: bewegliches Auslöseelement
- 51: Gleitabschnitt des Auslöseelements
- 52: Biegegelenk (bzw. Federgelenk oder Vorspannmittel)
- 54: Entsicherungsausnehmung im Auslöseelement
- 55: Drehbewegung des Auslöseelements (Pfeil)
- 56: Auslöseschlagbolzen
- 57: Bolzenhalterung
- 60: Sicherungselement
- 61: Stützfläche des Sicherungselements
- 62: Biegebewegung des Sicherungselements (Pfeil)
- 66: zweistufige Auslösesicherung
- 67: erstes Sicherungsmittel
- 68: zweites Sicherungsmittel
- 70: Indikatorelement
- 71: mechanisches Anzeigemittel
- 72: Farbanzeigemittel (bzw. Farbkapsel)
- 73: saugfähiges Material
- 74: elektronisches Anzeigemittel
- 75: Bewegungsrichtung des Indikatorelements
- 80: Vorspannmittel
- 81: Vorspannfeder
- 90: Gegengewicht
- 100: Baustoff
- 101: Oberfläche des Baustoffs
- 200: Schutzverpackung
- L₂₀: Länge des hygrischen Längenänderungselements bei 20% relativer Luftfeuchte
- L₇₉: Länge des hygrischen Längenänderungselements bei 79% relativer Luftfeuchte
- L₈₀: Länge des hygrischen Längenänderungselements bei 80% relativer Luftfeuchte
- L₈₅: Länge des hygrischen Längenänderungselements bei 85% relativer Luftfeuchte
- L₁₀₀: Länge des hygrischen Längenänderungselements bei 100% relativer Luftfeuchte
- RF₂₀: 20% relative Luftfeuchte
- RF₇₉: 79% relative Luftfeuchte; hier: unterschrittener Schwellwert
- RF₈₀: 80% relative Luftfeuchte; hier: Schwellwert
- RF₈₅: 85% relative Luftfeuchte
- RF₁₀₀: 100% relative Luftfeuchte

## Patentansprüche

1. Feuchteindikatorvorrichtung (1) zur hygrometrischen Bestimmung der Restfeuchte eines Baustoffs (100), umfassend ein Gehäuse (10) mit zumindest einem Gehäuseinnenraum (17), der über zumindest eine mit einer wasserdampfdurchlässigen Membran (16) bedeckten Diffusionsöffnung (15) mit der Gehäuseumgebung kommuniziert, wobei das Gehäuse (10) ein Anzeigefeld (13) aufweist, **dadurch gekennzeichnet, dass** im Gehäuseinnenraum (17) zumindest ein hygrisches Längenänderungselement (20) angeordnet ist, welches hygrische Längenänderungselement (20) abhängig von der im Gehäuseinnenraum (17) herrschenden relativen Luftfeuchte (RF₂₀, RF₇₉, RF₈₀, RF₈₅, RF₁₀₀) jeweils unterschiedliche Längen (L₂₀, L₇₉, L₈₀, L₈₅, L₁₀₀) einnimmt und mit einem mechanischen Auslösemechanismus (30) in Wirkverbindung steht, wobei bei Unterschreiten (RF₇₉) eines voreinstellbaren Schwellenwerts (RF₈₀) der relativen Luftfeuchte der mechanische Auslösemechanismus (30) in Abhängigkeit der eingenommenen Länge (L₇₉) des Längenänderungselements (20) im Anzeigefeld (13) ein Anzeigemittel (71, 72, 74) zur Anzeige der Schwellwertunterschreitung (RF₇₉) der voreingestellten relativen Luftfeuchte (RF₈₀) aktiviert.

2. Indikatorvorrichtung (1) nach Anspruch 1, **dadurch gekennzeichnet, dass** der mechanische Auslösemechanismus (30) zumindest ein beweglich (45) gelagertes Übertragungselement (40) sowie zumindest ein beweglich (55) gelagertes Auslöseelement (50, 56) umfasst, welche miteinander in Wirkverbindung stehen, wobei das Übertragungselement (40) mit dem hygrischen Längenänderungselement (20) bewegungsmäßig gekoppelt ist und Änderungen der jeweils eingenommenen Länge (L₂₀, L₇₉, L₈₀, L₈₅, L₁₀₀) des Längenänderungselements (20) dabei an das Auslöseelement (50, 56) überträgt, sowie das Auslöseelement (50, 56) bei Schwellwertunterschreitung (RF₇₉) der voreingestellten relativen Luftfeuchte (RF₈₀) das Anzeigemittel (71, 72, 74) auslöst.

3. Indikatorvorrichtung (1) nach Anspruch 1 oder 2, **dadurch gekennzeichnet, dass** der mechanische Auslösemechanismus (30) ein Sicherungselement (60) umfasst, welches Sicherungselement (60) von einer verriegelten Position (31) in eine entsicherte Position (32) des Auslösemechanismus (30) bewegbar (62) ist, wobei in der verriegelten Position (31) der Auslösemechanismus (30) auch bei einer Luftfeuchte (RF₂₀) unterhalb des Schwellwerts (RF₈₀) der voreingestellten relativen Luftfeuchte deaktiviert ist.

4. Indikatorvorrichtung (1) nach Anspruch 2 oder 3, **dadurch gekennzeichnet, dass** das zumindest eine Übertragungselement (40) an einer in Längsachsenrichtung (22) des hygrischen Längenänderungselements (20) angeordneten freien Endfläche (24) mit diesem bewegungsmäßig gekoppelt ist, wobei das hygrische Längenänderungselement (20) an seiner in Längsachsenrichtung (22) entgegengesetzt gelegenen freien Endfläche (23) am Gehäuse (10), vorzugsweise mit einem Festlager (27), gelagert ist.

5. Indikatorvorrichtung (1) nach einem der Ansprüche 1 bis 4, **dadurch gekennzeichnet, dass** zum Einstellen des Schwellwerts (RF₈₀) der bei einer definierten relativen Luftfeuchte eingenommenen Länge (L₈₀) des Längenänderungselements (20) die Lagerposition (27) des hygrischen Längenänderungselements (20) im Gehäuseinnenraum (17) mittels eines Justiermechanismus (28), vorzugsweise mit einer Justierschraube (28), justierbar ist.

6. Indikatorvorrichtung (1) nach einem der Ansprüche 2 bis 5, **dadurch gekennzeichnet, dass** das Übertragungselement (40) ein Haltemittel (41) umfasst, welches Haltemittel (41) abhängig von der jeweils eingenommenen Länge (L₈₀, L₈₅, L₁₀₀) des Längenänderungselements (20) bei einer Luftfeuchte oberhalb der voreingestellten relativen Luftfeuchte (RF₈₀) entlang eines bahnförmigen Gleitabschnitts (51) des Auslöseelements (50) gleitet, wobei das Haltemittel (41) bei Unterschreiten (RF₇₉) des Schwellwerts (RF₈₀) der voreingestellten relativen Luftfeuchte den bahnförmigen Gleitabschnitt (51) des Auslöseelements (50) verlässt, wodurch die Stützfunktion des Haltemittels (41) für das Auslöseelement (50) endet und das Auslöseelement (50) aus seiner Ruhelage auslöst (55).

7. Indikatorvorrichtung (1) nach einem der Ansprüche 3 bis 6, **dadurch gekennzeichnet, dass** das Sicherungselement (60) auf ein im Wesentlichen bügelförmiges Auslöseelement (50) wirkt und eine Stützfläche (61) umfasst, welche Stützfläche (61) abhängig von der jeweils eingenommenen Länge (L₈₀, L₈₅, L₁₀₀) des Längenänderungselements (20) bei einer ansteigenden Luftfeuchte oberhalb der voreingestellten relativen Luftfeuchte (RF₈₀) entlang des bahnförmigen Gleitabschnitts (51) des Auslöseelements (50) gleitet, bis die Stützfläche (61) des Sicherungselements (60) in eine Entsicherungsausnehmung (54) des Auslöseelements (50) eingreift und dabei das Haltemittel (41) anstelle der Stützfläche (61) die Stützfunktion des Auslöseelements (50) übernimmt.

8. Indikatorvorrichtung (1) nach einem der Ansprüche 3 bis 6, **dadurch gekennzeichnet, dass** das Sicherungselement (60) eine zweistufige Auslösesicherung (66) mit einem ersten Sicherungsmittel (67) und einem zweiten Sicherungsmittel (68) umfasst, welche Sicherungsmittel (67, 68) auf eine Bolzenhalterung (57) eines im Wesentlichen bolzenförmigen Auslöseelements (56) wirken, wobei in einer verriegelten Position (31) des Auslösemechanismus (30) das erste Sicherungsmittel (67) in die Bolzenhalterung (57) eingreift und in einer entsicherten Position (32) des Auslösemechanismus (30) abhängig von der jeweils eingenommenen Länge (L₈₀, L₈₅, L₁₀₀) des Längenänderungselements (20) bei einer Luftfeuchte oberhalb der voreingestellten relativen Luftfeuchte (RF₈₀) solange das zweite Sicherungsmittel (68) in die Bolzenhalterung (57) eingreift, bis bei Unterschreiten (RF₇₉) des Schwellwerts (RF₈₀) der voreingestellten relativen Luftfeuchte aufgrund der korrespondierenden eingenommenen Länge (L₇₉) des Längenänderungselements (20) das zweite Sicherungsmittel (68) die Bolzenhalterung (57) freigibt.

9. Indikatorvorrichtung (1) nach einem der Ansprüche 1 bis 8, **dadurch gekennzeichnet, dass** das hygrische Längenänderungselement (20) ausgewählt ist aus einer Gruppe umfassend:
- Holz (21), vorzugsweise Buchenholz;
- Naturhaar menschlichen oder tierischen Ursprungs;
- Papier und/oder Karton;
- Kunststoff, vorzugsweise Polyamid
- Absorberelement (29), befüllt mit Superabsorber;
- Absorberelement (29), befüllt mit hygrischem Mineralstoff oder hygrischem Mineralstoffgemenge, vorzugsweise mit Bentonit und/oder Montmorillonit.

10. Indikatorvorrichtung (1) nach einem der Ansprüche 1 bis 9, **dadurch gekennzeichnet, dass** das Anzeigemittel (71, 72, 74) zur Anzeige der Schwellwertunterschreitung der voreingestellten relativen Luftfeuchte (RF₈₀) ausgewählt ist aus einer Gruppe umfassend:
- mechanisches Anzeigemittel (71);
- Farbanzeigemittel (72);
- elektromechanisches Anzeigemittel;
- elektronisches Anzeigemittel (74).

## Claims

1. Moisture indicator apparatus (1) for the hygrometric determination of the residual moisture of a construction material (100), comprising a housing (10) with at least one housing interior space (17), which communicates with the housing's surrounding environment via at least one diffusion opening (15) covered with a water-vapour-permeable membrane (16), wherein the housing (10) comprises a display field (13), **characterized in that** at least one hygric length change element (20) is arranged in the housing interior space (17), which hygric length change element (20) respectively assumes different lengths (L₂₀, L₇₉, L₈₀, L₈₅, L₁₀₀) depending on the relative air moisture (RF₂₀, RF₇₉, RF₈₀, RF₈₅, RF₁₀₀) prevailing in the housing interior space (17) and is operatively connected to a mechanical release mechanism (30), wherein, in the event of undercutting (RF₇₉) a presettable threshold value (RF₈₀) for air moisture, the mechanical release mechanism (30) activates a display means (71, 72, 74) in the display field (13) for displaying the threshold value undercut (RF₇₉) of the preset relative air moisture (RF₈₀) depending on the length (L₇₉) of the length change element (20) assumed.

2. Indicator apparatus (1) according to Claim 1, **characterized in that** the mechanical release mechanism (30) comprises at least one moveably (45) mounted transmission element (40), as well as at least one moveably (55) mounted release element (50, 56), which are operatively connected to each other, wherein the transmission element (40) is moveably coupled to the hygric length change element (20), thereby transmitting changes in the respectively assumed length (L₂₀, L₇₉, L₈₀, L₈₅, L₁₀₀) of the length change element (20) to the release element (50, 56), and the release element (50, 56) triggers the display means (71, 72, 74) in the event of the preset threshold value (RF₇₉) for the relative air moisture (RF₈₀) being undercut.

3. Indicator apparatus (1) according to Claim 1 or 2, **characterized in that** the mechanical release mechanism (30) comprises a locking element (60), which locking element (60) can be moved (62) from a locked position (31) into an unlocked position (32) of the release mechanism (30), wherein, in the locked position (31), the release mechanism (30) is deactivated even in the event of an air moisture (RF₂₀) being under the threshold value (RF₈₀) for the preset relative air moisture.

4. Indicator apparatus (1) according to Claim 2 or 3, **characterized in that,** on a free end face (24) arranged in the longitudinal axis direction (22) of the hygric length change element (20), the at least one transmission element (40) is moveably coupled to this, wherein the hygric length change element (20) is mounted to the housing (10) on its free end face (23) situated in a manner opposing the longitudinal axis direction (22), preferably being mounted to a fixed bearing (27).

5. Indicator apparatus (1) according to one of the Claims 1 to 4, **characterized in that,** in order to set the threshold value (RF₈₀) of the length (L₈₀) of the length change element (20) assumed in the case of a defined relative air moisture, the bearing position (27) of the hygric length change element (20) can be adjusted in the housing interior space (17) by means of an adjustment mechanism (28), preferably using an adjustment screw (28).

6. Indicator apparatus (1) according to one of the Claims 2 to 5, **characterized in that** the transmission element (40) comprises a retaining means (41), which retaining means (41) slides along a track-shaped sliding section (51) of the release element (50) in the case of an air moisture above the preset relative air moisture (RF₈₀) depending on the respectively assumed length (L₈₀, L₈₅, L₁₀₀) of the length change element (20), wherein the retaining means (41) exits the track-shaped sliding section (51) of the release element (50) in the event the threshold value (RF₈₀) for the preset relative moisture is undercut (RF₇₉), wherein the support function of the retaining means (41) for the release element (50) ends and the release element (50) releases (55) from its resting position.

7. Indicator apparatus (1) according to one of the Claims 3 to 6, **characterized in that** the locking element (60) acts on an essentially bow-shaped release element (50) and comprises a support surface (61), which support surface (61) slides along the track-shaped sliding section (51) of the release element (50) in the event of an increasing air moisture within the preset relative moisture (RF₈₀) depending on the respectively assumed length (L₈₀, L₈₅, L₁₀₀) of the length change element (20) until the support surface (61) of the locking element (60) engages into an unlock recess (54) of the release element (50) and the retaining means (41) thereby assumes the support function of the release element (50) instead of the support surface (61).

8. Indicator apparatus (1) according to one of the Claims 3 to 6, **characterized in that** the locking element (60) comprises a two-level anti-release safety device (66) with a first locking means (67) and a second locking means (68), which locking means (67, 68) act on a bolt holder (57) of an essentially bolt-shaped release element (56), wherein, in a locked position (31) of the release mechanism (30), the first locking means (67) engages into the bolt holder (57) and, in an unlocked position (32) of the release mechanism (30), depending on the assumed length (L₈₀, L₈₅, L₁₀₀) of the length change element (20) in the event of an air moisture (RF₈₀) being above the preset relative air moisture (RF₈₀), while the second locking means (68) engages into the bolt holder (57) until the second locking means (68) releases the bolt holder (57) in the case of undershooting (RF₇₉) the threshold value (RF₈₀) for the preset relative air moisture due to the correspondingly assumed length of the length change element (20).

9. Indicator apparatus (1) according to one of the Claims 1 to 8, **characterized in that** the hygric length change element (20) is selected from a group comprising:
- wood (21), preferably beechwood;
- natural hair deriving from human or animal origin;
- paper and/or cardboard;
- plastic, preferably polyamide;
- absorber element (29), filled with super absorber;
- absorber element (29), filled with a hygric mineral material or hygric material mixture, preferably with bentonite and/or montmorillonite.

10. Indicator apparatus (1) according to one of the Claims 1 to 9, **characterized in that** the display means (71, 72, 74) for displaying the threshold value undercut of the preset relative air moisture (RF₈₀) is selected from a group comprising:
- mechanical display means (71);
- colour display means (72);
- electromechanical display means;
- electronic display means (74).

## Revendications

1. Dispositif indicateur d'humidité (1) pour la détermination hygrométrique de l'humidité résiduelle d'un matériau de construction (100), comprenant un boîtier (10) comportant au moins un espace intérieur de boîtier (17) qui communique par au moins un orifice de diffusion (15) recouvert par une membrane perméable à la vapeur d'eau (16) avec l'environnement du boîtier, le boîtier (10) présentant un champ d'affichage (13), **caractérisé en ce que,** dans l'espace intérieur du boîtier (17), est disposé au moins un élément de modification de longueur hygrique (20), lequel élément de modification de longueur hygrique (20) comprend, en fonction de l'hygrométrie relative (RF₂₀, RF₇₉, RF₈₀, RF₈₅, RF₁₀₀) régnant dans l'espace intérieur de boîtier (17), adopte respectivement différentes longueurs (L₂₀, L₇₉, L₈₀, L₈₅, L₁₀₀) et est en liaison fonctionnelle avec un mécanisme de déclenchement (30), dans lequel, en cas de non-atteinte (RF₇₉) d'une valeur seuil préréglable (RF₈₀) de l'hygrométrie relative, le mécanisme de déclenchement (30), en fonction de la longueur adoptée (L₇₉) par l'élément de modification de longueur (20), dans le champ d'affichage (13) active un moyen d'affichage (71, 72, 74) servant à afficher la non-atteinte (RF₇₉) de la valeur seuil préréglée d'hygrométrie relative (RF₈₀).

2. Dispositif indicateur (1) selon la revendication 1, **caractérisé en ce que** le mécanisme de déclenchement mécanique (30) comprend au moins un élément de transfert (40) à palier mobile (45) et au moins un élément de déclenchement (50, 56) à palier mobile (55) qui sont en liaison fonctionnelle l'un avec l'autre, l'élément de transfert (40) étant couplé à l'élément de modification de longueur hygrique (20) et transférant alors les modifications de la longueur respectivement adoptée (L₂₀, L₇₉, L₈₀, L₈₅, L₁₀₀) par l'élément de modification de longueur (20) à l'élément de déclenchement (50, 56), et l'élément de déclenchement (50, 56), en cas de non-atteinte de la valeur seuil (RF₇₉) de l'hygrométrie relative préréglée (RF₈₀), déclenchant le moyen d'affichage (71, 72, 74).

3. Dispositif indicateur (1) selon la revendication 1 ou 2, **caractérisé en ce que** le mécanisme de déclenchement mécanique (30) comprend un élément de blocage (60), lequel élément de blocage (60) est mobile (62) d'une position verrouillée (31) vers une position débloquée (32) du mécanisme de déclenchement (30), dans lequel, dans la position verrouillée, le mécanisme de déclenchement (30) est désactivé même en cas d'hygrométrie (RF₂₀) en-dessous de la valeur seuil (RF₈₀) de l'hygrométrie relative préréglée.

4. Dispositif indicateur (1) selon la revendication 2 ou 3, **caractérisé en ce que** l'au moins un élément de transfert (40) est couplé à une surface terminale libre (24) disposée dans le sens de l'axe longitudinal (22) de l'élément de modification de longueur hygrique (20) à celui-ci au niveau du mouvement, l'élément de modification de longueur hygrique (20) s'appuyant, au niveau de sa surface terminale libre (23) située en sens opposé dans le sens de l'axe longitudinal (22), au niveau du boîtier (10), de préférence par un palier fixe (27).

5. Dispositif indicateur (1) selon une des revendications 1 à 4, **caractérisé en ce que** pour régler la valeur seuil (RF₈₀) de la longueur (L₈₀) adoptée avec une hygrométrie relative définie de l'élément de modification de longueur (20), la position d'appui (27) de l'élément de modification de longueur hygrique (20) dans l'espace intérieur du boîtier (17) est réglable au moyen d'un mécanisme de réglage (28), de préférence d'une vis de réglage (28).

6. Dispositif indicateur (1) selon une des revendications 2 à 5, **caractérisé en ce que** l'élément de transfert (40) comprend un moyen de retenue (41), lequel moyen de retenue (41), en fonction de la longueur respectivement adoptée (L₈₀, L₈₅, L₁₀₀) par l'élément de modification de longueur (20), dans le cas d'une hygrométrie supérieure à l'hygrométrie relative préréglée (RF₈₀), glisse le long d'une section de glissement en forme de bande (51) de l'élément de déclenchement (50), le moyen de retenue (41), en cas de non-atteinte (RF₇₉) de la valeur seuil (RF₈₀) de l'hygrométrie relative préréglée, quitte la section de glissement en forme de bande (51) de l'élément de déclenchement (50), ce qui met fin à la fonction de soutien du moyen de retenue (41) pour l'élément de déclenchement (50) et fait sortir l'élément de déclenchement (50) de sa position de repos (55).

7. Dispositif indicateur (1) selon une des revendications 3 à 6, **caractérisé en ce que** l'élément de blocage (60) agit sur un élément de déclenchement (50) sensiblement en forme d'étrier et comprend une surface de soutien (61), laquelle surface de soutien (61), en fonction de la longueur respectivement adoptée (L₈₀, L₈₅, L₁₀₀) par l'élément de modification de longueur (20), en cas d'hygrométrie augmentant au-delà de l'hygrométrie relative préréglée (RF₈₀) glisse le long de la section de glissement en forme de bande (51) de l'élément de déclenchement (50) jusqu'à ce que la surface de soutien (61) de l'élément de blocage (60) s'engrène dans une échancrure de déblocage (54) de l'élément de déclenchement (50) et que l'élément de retenue (41) prenne en charge, à la place de la surface de soutien (61), la fonction de soutien de l'élément de déclenchement (50).

8. Dispositif indicateur (1) selon une des revendications 3 à 6, **caractérisé en ce que** l'élément de blocage (60) comprend un blocage de déclenchement à deux niveaux (66) comportant un premier moyen de blocage (67) et un second moyen de blocage (68), lesquels moyens de blocage (67, 68) agissent sur un support de goujon (57) d'un élément de déclenchement sensiblement en forme de goujon (56), dans lequel, dans une position verrouillée (31) du mécanisme de déclenchement (30), le premier moyen de blocage (67) s'engrène dans le support de goujon (57) et, dans une position déverrouillée (32) du mécanisme de déclenchement (30), en fonction de la longueur respectivement adoptée (L₈₀, L₈₅, L₁₀₀) par l'élément de modification de longueur (20), dans le cas d'une hygrométrie relative supérieure à l'hygrométrie relative préréglée (RF₈₀), le second élément de blocage (68) s'engrène dans le support de goujon (57) jusqu'à ce que, en cas de non-atteinte (RF₇₉) de la valeur seuil (RF₈₀) de l'hygrométrie relative préréglée, en raison de la longueur correspondante adoptée (L₇₉) par l'élément de modification de longueur (20), le second moyen de blocage (68) débloque le support de goujon (57).

9. Dispositif indicateur (1) selon une des revendications 1 à 8, **caractérisé en ce que** l'élément de modification de longueur hygrique (20) est sélectionné dans le groupe comprenant :
- du bois (21), de préférence du bois de hêtre ;
- des poils naturels d'origine humaine ou animale ;
- du papier et/ou du carton ;
- de la matière plastique, de préférence du polyamide ;
- un élément absorbant (29) rempli de super-absorbant ;
- un élément absorbant (29) rempli de substances minérales hygriques ou d'un mélange de substances minérales hygriques, comprenant de préférence de la bentonite et/ou de la montmorillonite.

10. Dispositif indicateur (1) selon une des revendications 1 à 9, **caractérisé en ce que** le moyen d'affichage (71, 72, 74) servant à afficher la non-atteinte de valeur seuil de l'hygrométrie relative préréglée (RF₈₀) est sélectionné dans un groupe comprenant :
- un moyen d'affichage mécanique (71) ;
- un moyen d'affichage de couleur (72) ;
- un moyen d'affichage électromécanique ;
- un moyen d'affichage électronique (74).
